(19) Europäisches Patentamt
European Patent Office
Office européen des brevets

(11) EP 1 371 408 A1

(12) # EUROPEAN PATENT APPLICATION

published in accordance with Art. 158(3) EPC

(43) Date of publication:
**17.12.2003 Bulletin 2003/51**

(21) Application number: **00977953.9**

(22) Date of filing: **27.11.2000**

(51) Int Cl.[7]: **B01D 53/86**, A61L 9/00, A61L 9/18

(86) International application number:
**PCT/JP00/08340**

(87) International publication number:
**WO 02/043839 (06.06.2002 Gazette 2002/23)**

(84) Designated Contracting States:
**DE ES FR GB IT NL SE TR**

(71) Applicant: **Toyo ELement Industry Co., Ltd.**
**Kawasaki-shi, Kanagawa 216-0015 (JP)**

(72) Inventor: **INOUE, Kiyoshi**
**Setagaya-ku, Tokyo 158-0098 (JP)**

(74) Representative: **Urizar Anasagasti, José Antonio**
**Puerto Rico 6A, Bajo**
**28016 Madrid (ES)**

(54) **ENERGY SAVING AIR CLEANER**

(57) It has been hitherto impossible to continuously and constantly clean or purify a residential environment in a stable manner, while toxic materials in indoor air are decomposed little by little.

According to the present invention, there is provided an air purifier in which a catalyst is activated under low energy' necessary electric power is minimized, a continuous operation can be performed and the residential environment can be constantly cleaned in a stable manner, while the toxic materials in the indoor air are decomposed little by little.



Printed by Jouve, 75001 PARIS (FR)

## Description

### Technical Field

The present invention relates to an apparatus for purifying indoor air mainly in a living room or an automobile, or air in a city or town, and more particularly to an apparatus which uses an optically activated catalyst containing titanium oxide as a main component and activated by noble metal to decompose and detoxify toxic materials in the air.

### Background of the invention

Generally, outside air at the intersections of the streets of a city with traffic congestion, or air in a residential section near roads is contaminated with a lot of organic and inorganic toxic materials. For instance, main materials of them are described below...

Ozone $O_3$: 300ppb to 500ppb
Carbon monoxide CO: 5,000ppb to 20ppm
Olefin $C_nH_{2n}$ (n = 2 to 20): 400ppb to 3000ppb
Nitrogen oxide $NO_x$: 500ppb to 10ppm

Further, the following materials are detected on the order of 20ppb to 50ppb.
Aldehyde R-CHO
Ether $CH_2$-O-$CH_2$
Butylene $C_4H_8$
Ketone RCOR
Methane $CH_4$
Methyl alcohol: $CH_3OH$
Ethylene -$H_2$-

Since these materials are harmful in view of health, and cause various kinds of allergic diseases, asthma, lung cancer and the like, they need to be detoxified, however, an air purifier hitherto supplied for the people's livelihood has been a type that simply captures airborne dust in air. A device that needs a large amount of electric energy or a device that utilizes a solar light and the like has been proposed as an air purifier or cleaner which can decompose these toxic gases and detoxify them, however, a device that can treat the toxic gases under low energy except them has not been proposed.

A purpose of the present invention resides in the provision of an inexpensive and safe air purifier for the people's livelihood which can decompose toxic materials in the air of a living room of persons or a working place and can be used to improve the environment of a living space.

As catalysts generally utilized for industrial use or waste or exhaust gases of vehicles for the purpose of removing the above-described toxic gases, there have been known Au, Pa, Pt, Rn, Ru, Ti, Mn, Cu, W, Al, Zr, Ce, Tb, Sm, Er, La, and Dy, and CuO, $TiO_2$, $WO_3$, $Mn_2O_3$, $Mn_8O_{16}$, $Al_2O_3$, $ZrO_2$, $MnO_2$, $Ce_2O_3$, $Tb_2O_3$, $Sm_2O_3$, $Er_2O_3$, $La_2O_3$, $Dy_2O_3$ and the like as oxides thereof. These materials are used independently, or in combination to the end of use, and further, are activated by electromagnetic wave as required and then employed.

As an especially useful technique, the photocatalyst of $TiO_2$ was disclosed in 1972 and a photocatalyst activated by noble metal was disclosed in 1979. As the applied technology thereof, the simultaneous use of the photocatayst and solid carbons was disclosed in 1980, the decomposition of carbohydrate was published in 1980 and the decomposition of protein and fatty high polymers were respectively published in 1981.

Specially, an activation method for a photocatryst by metal particles having the size within a range of 1 to 10 nm and various kinds of production methods of $TiO_2$ have been also well known. There have been also disclosed combinations of a photocatalyst with Ag or other rare earth elements, and further with $WO_3$, $Fe_2O_3$, CdS, MoS and the like. All of them were the technology well known in 1980s.

### Disclosure of the Invention

An energy saving type air purifier according to the present invention comprises al Catalyst containing titanium oxide ahs a main component and activated by noble metal, a low electric power light source for optically activating the catalyst and a power source with a small capacity for emitting the light from the light source.
The area of the catalyst is not especially limited, however, it is ordinarily set to 500 $cm^2$ or larger and 10,000 $cm^2$ or smaller, and desirably, to 1,000 $cm^2$ or larger and 5,000 $cm^2$ or smaller. The driving electric power of the light source is 10 $\mu$W or higher and 15 W or lower per 1 $cm^2$ of the catalyst and desirably 10$\mu$W or higher and 100$\mu$W or lower per

1 $cm^2$ of the catalyst.

When the area of the catalyst is 1,000 $cm^2$ or smaller, a purification capability is excessively decreased. However, when the area of the catalyst is 10,000 $cm^2$ or larger, the thickness of a sheet required for ordinarily obtaining a desired strength is excessively increased. When the air of large volume is to be cleaned, a plurality of devices of the above size are more preferably used than one device of large capacity. Because when the device is enlarged, the inner capacity of the device is too increased relatively to the area of the catalyst, which causes the contact efficiency of the catalyst to be relatively deteriorated. Further, also when a granular catalyst is employed, if the size of the device is enlarged more than the above-described size, air resistance will be increased. Therefore, the size of the device is preferably restricted to the above-described size.

Further, when the driving electric power of the light source is 10 μW or lower per $cm^2$ of the catalyst, treatment capability inappropriately excessively deteriorated. Even when the driving electric power of the light source is 15 mW or higher, the concentration of toxic materials is not so lowered and necessary electric power is wasted.

As the light source, a semiconductor light emitting element such as LED, LSD and LD is recommended to be used. Because, when other light sources are employed by turning on and off them, the life span of them is shortened. Even when the semiconductor light s6urc6'is used under the above-described using conditions, its life span is extremely long, its wavelength is proper, its efficiency is high and the light emission control of high frequency can be easily performed.

<u>Best Mode for Carring Out the invention</u>

Now, a catalyst used herein will be described below.

For instance, the decomposition reaction of a photocatalyst such as $TiO_2$ is generated by irradiating a part betweeh bahd gaps of a semiconductor with electromagnetic wave so that the oxidation potential and the reduction potential of a molecule oxidized and decomposed are present therein.

Thus, electrons of atoms in the valence electronic band of the catalyst are excited and moved to a conduction band and holes are left in the part of the valence electronic band. Then, pairs of electrons and holes are generated in the catalyst. The electrons and the holes move in the semiconductor $TiO_2$ so that the electrons exist as ($O_2$-) (O-) to become oxide ions and hydroxide radicals ($OH^+$) at the same time, serve as a reduction action and strong oxides, directly react with organic materials and react with C-C, C-H, C-Cl, H-Cl and the like of the organic materials and generate the decomposition reaction.

A series of reactions are generated as described above and a user needs to pay his attention to the following points.

1. The problem of the defect of a lattice plane of a $TiO_2$ Catalyst
2. The problem of the defects of many lattice points of a $TiO_2$ Catalyst
3. The problem of irregularity of crystal size of a $TiO_2$ Catalyst
4. The problem of recombination of electrons and holes.

Here, if the electrons are recombined with the holes, the decomposition action will never be generated. Thus, the $TiO_2$ Catalyst becomes a catalyst having an extremely degraded efficiency.

It is necessary to simultaneously generate both oxidation and reduction actions in one catalyst particle. This seems to be contradictory at a glance, however, the problems of the above items 1 to 3 can be solved by obtaining. a fine catalyst $TiO_2$ with the particle size of 0.1 to 10 nm and the defects can be prevented.

The above-described matter can be realized by forming a perfectly crystallized size. For example, also when the catalyst particle is deposited by a hydrolytic operation, decomposition speed is accurately controlled so that the perfectly crystallized size can be obtained.

Initially, $TiC_4$ of 0.025 mol/liter including poly-nuclear complex ion liquid having chloride ions of Ti as a starting material is subject to a hydrolytic decomposition process for 24 hours at 70°C and urea is added to the resultant product uhder pH of 6.5 and the urea added product is subject to a condensation polymerization to form a colloidal nucleus. The shape of a particle is an anatase of a cubic particle and the average particle size is 10 nm (in accordance with an image by an electron microscope). A strong interaction is generated between particles, however, the condensation of primary particles including chlorine/ mainly serves to maintain the balance between force to reduce energy on the particle and interfacial tension to form a particle.

Therefore, pH of final strong liquid in which the cubic particle anatase is included the concentration of titanium chloride is lowered and production temperature is raised needs to range from 1 to 2. Under these conditions, a single-nucleus hydroxo- complex Ti-O-Ti is formed, washed with water, dried and ground to obtain fine $TiO_2$ particles.

$K_2PtC_6$ was suspended in methanol aqueous solution so that $TiO_2$ and Pt were in the weight ratio 1:1. While the suspension was irradiated with the light of wavelength of 276 nm, the suspension was irradiated with ultrasonic

wave (0.1 W/$cm^2$) of 100kHz and 60 W as a stirrer. The suspension was strongly agitated and treated to deposit Pt on the surface of the particle composed of titanium oxide.

Consequently, Pt was deposited on the $TiO_2$ particle with the particle size of 10 nm to obtain a $TiO_2$Pt catalyst. The size of Pt was 1nm n the order of about 1/10 as large as that of $iO_2$. About 10 pieces of Pt particles could be stuck to the surface of the $TiO_2$ particle.

The catalyst particle was kneaded with water again to extrude from a nozzle with an aperture of 1 mm, the extruded product was cut to 1 mm to obtain pellets and the pellets were dried and then burnt at 300°C for 3 hours, hence a granular titanium oxide catalyst having a surface area of approximately 300 to 400 $m^2$/g could be obtained.

Although the material to be treated is produced in a granular form in order to make the fluidity thereof good, it is to be understood that the material may be produced in a film form, a plate form, a scale form and the like. Further, the mixed crystal of $TiO_2$ and $SiO_2$ may be employed or the photochemical excitation effect of a rare earth element may be freely employed.

Finally stated, the matter of the combination of electrons and holes in the crystal contributes an extremely important performance as the catalyst It is to be realized that a reaction holding time changes depending on the depth of the reaction. With this fact taken into consideration, assuming that a reaction product generated on the surface of the catalyst is not supplied from its inner part and the emission rate of electrons and holes is Q when the surface of the catalyst is exposed to electromagnetic wave, the following formula 1 is established.

$$Q=d\sigma/dt=\sigma/\tau \quad (1)$$

σ: density of electrons and holes on the surface,
t: time
τ:Staying or detention time

Here, assuming that τ is constant, the following formula 2 is established.

$$Q(t)=\sigma/\tau\exp(-t/\tau) \quad (2)$$

t: lapse of time

The above formula represents that the emission rate of electrons and holes are reduced in accordance with a time constant.

Actually, when the emission layers of electrons and holes are composed of many layers, a space of a solid surface exposed to the electromagnetic wave becomes different. Since the activating energy of the catalyst changes dependent on an amount of emission, the emission of the electrons and holes may be described by the following formula 3.

$$Q \ 1/t \quad (3)$$

Therefore, with the oscillation of molecules taken into account, assuming that a time during which the electrons and holes stay on the surface is $10^{-2}$ to $10^{-3}$ on the average, which is greatly different depending on the production state of a crystal, the following formula 4 is established.

$$\tau=\tau_0\exp(E/RT) \quad (4)$$

$\tau_0$: Cycle during which surface atoms generally oscillate in a potential depth E for time of about $10^{-11}$ seconds
E: potential depth

Accordingly, when temperature rises from 20°C 100°C, the staying time of the electrons and holes may be estimated to range approximately from $10^{-1}$ seconds to $10^{-3}$ lo $10^{-4}$ seconds, so that the repeated cycles of photochemical excitation is recommended to be changed so as to meet the temperature and the detention or staying time of the electrons and holes.

A reason why the irradiation of electromagnetic wave is carried out in a pulsating manner is that an operation

is made under lower electric power. Since the desired repeated frequency of pulsating irradiation is dependent on temperature T, the repeated frequency is desirably efficiently controlled as the function of the temperature.

The semiconductor light emitting element such as LED, SLD and LD is advantageously employed to selectively control the repeated frequency of the irradiation of electromagnetic wave, especially, the LED having the wavelength of about 400 to 430 nm is advantageous. The output efficiency of the electromagnetic wave is 91 cd/W for a fluorescent lamp with high luminance. As compared with the above, in the case of the LED, the output efficiency of the electromagnetic wave is 300cd/W which is increased to about three times as high as that of the fluorescent lamp. Additionally, when the electrons and holes are always present under the pulses with a duty factor of about 10%, that is, an on/off ratio 1:9, the output efficiency of the electromagnetic wave for the LED becomes 3000 cd/W which is approximately 30 times as high as that of the former, so that the efficiency is outstandingly improved. Besides, the pulsating irradiation of the electromagnetic makes it possible to further improve an electromagnetic wave density. As described above, according to the present invention, the efficiency 30 times or more as high as the related art can be obtained.

As for deodorization, assuming that the intensity of odor is I and the concentration of an offensive material is Co, the following formula 5 is established.

$$I \log C_0 \tag{5}$$

Accordingly, a catalytic operation needs to be logarithmically carried out for deodorization.

## Example 1

A tubular catalyst case having both ends opened is manufactured by an aluminum pipe having a mesh type wall surface or a square type pipe. An LED is attached to the inner surface of the pipe. The catalyst case is allowed to stand at the corner of a room. The catalyst is excited to circulate in convection contaminated air in the room through the case by its draft action. When outside is light, a purification action is carried out outside.

Therefore, the opening on the upper end of the case is located at a position lower by about 30 cm than a ceiling and the lower end thereof is arranged so that an inlet port is located at a position higher by about 50 cm than a floor surface. Occasionally, a bimorph type fan of approximately 0.05 W for circulating air may be inserted into the case.

$TiO_2PtEr$ catalyst was applied to the inner surface of the tubular case composed of the mesh type wall having inside diameter of 10cm and length of 1.5 m at the rate of 15g relative to 3000 $cm^2$. 8 LEDs having wavelength of 430 nm and 3cd were arranged in the case to emit light in a pulsating manner.

The tubular case was vertically installed in the room having the floor surface of 10 m × 5 m and the height of the ceiling of 2.3 m. Electric current having on-time of 0.8 msec and off-time of 10 msec, 3.1 V and a peak value of 42mA was supplied to emit light from the LEDs in a pulsating manner.

In the case, a fan of 3.2V and 0.03W was inserted and driven by a lithium sgcondary battery with a capacity of 12.6AH to supply indoor air of 0.5 $m^3$/min and circulate it. NOx was especially carefully measured, and it generated a reaction and was decomposed.

$hvH^2O$

$NO_x \quad NO_2 \quad NO_3 \text{---} \; HNO_3$

$TiO\ _2PtEr$

As a result of measurement, the concentration of $NO_x$ at the inlet of the case, which was 11 ppm, was lowered to 7 ppm at an outlet. After a continuous operation for about 41 hours, the average concentration of $NO_x$ in all the room was 1.1 ppm.

Further, as for the removal of olefin, $0_3$ and the like, it was found that tile final concentration thereof was about 10% or lower of an initial concentration. Average room temperature was 26 °C or so and humidity was 60%.

The room faces an intersection at which the number of vehicles passing a day was 140,000 on the average and four persons ordinarily lived there and freely got in and out.

The fan and the catalyst case were continuously driven for 10 days by the lithium secondary battery above description without recharging. When a solar cell serving as an auxiliary power source was used at the same time, the

fan and the case could be continuously used for one month.

In both cases, it was proved similarly to the above that the toxic materials could be removed so that the concentration thereof is lowered up to about 1/10 of the initial concentration.

## Example 2

The above described fine particle catalyst was blended in porous fluorine resin in the weight ratio of 50%, they were kneaded and rolled to form a sheet with the thickness of 0.1 mm. When the sheet of 1000 $cm^2$ was formed and attached to a position of 5cm spaced from an outdoor lamp so that the catalyst was activated by the light of the outdoor lamp lighted at night and solar light during the daytime, nitric add of 0.42 $\mu mol/cm^2$ h was detected on the average of one day and night.

In the cast of the catalyst for the outdoor lamp, it can be used in express-highways, city roads in traffic congestion areas, inside a tunnel and the like without a power source, and the solar cell may be advantageously used at the same time. The catalyst may be effectively built in the noise reduction wall of a road.

The catalyst may be attached to the reflecting plate of an indoor electric lamp and a fluorescent lamp without using a power source. In this case, it is to be understood that the solar cell may be effectively used at the same time.

The form of the catalyst may be arbitrarily designed. Further, a meter for measuring an electric conductivity, an automatically lighting switch, a buzzer, and the like are attached to the catalyst to detect a reaction state so that the catalyst can be more advantageously employed.

This catalyst has a decomposition effect for dioxins and the dioxins can be decomposed into chlorides, borides and oxides.

Further, the catalyst is also effective for CO gas due to imperfect combustion. In this case, exhaust gas is effectively subject to a washing treatment with water in combination with the catalyst In addition, the catalyst may be used as an anti-toxic mask and a sterilization function and a deodorization function may be obtained in a hospital and the like.

They can be continuously driven for one month or more even by a small battery, so that they are extremely advantageous.

## Industrial Applicability

The present invention has a great effect and a wide range of application as an apparatus which can highly efficiently decompose organic materials and the like under extremely low energy, decompose toxic materials indoor and outdoor air little by little and continuously purify the air.

## Claims

**1.** An energy saving type air purifier comprising a catalyst containing titanium oxide as a main component and activated by noble metal or the like, a low electric power light source for applying a photochemical activation to the catalyst and a power source for emitting light from the light source.

**2.** An air purifier according to claim 1 **characterized in that** the driving electric power of the light source is at least 10 $\mu W$ and at most 15mW per 1 $cm^2$ of the catalyst.

**3.** An air purifier according to claim 1 or 2 **characterized in that** the light source is composed of a semiconductor light emitting element.

**4.** An air purifier according to any one of claims 1 to 3 **characterized in that** the power source is a pulse power source.

**5.** An air purifier according to any one of claims 1 to 4 **characterized in that** the repeated frequency of the pulses of the power source is changed by temperature.

**6.** An air purifier according to any one of claims 1 to 5 **characterized in that** the catalyst is formed in a tubular form with both ends opened, the light source is arranged in the tubular catalyst and the tubular catalyst is disposed so as to generate a convection inside and outside the tubular catalyst.

**7.** An air purifier according to claim 6 **characterized in that** a low energy fan is provided in the tubular catalyst.

8. An air purifier according to claim 6 or 7 **characterized in that** a fitter is provided in the air inlet port of the tubular catalyst.

9. An air purifier according to any one of claims 1 to 8 **characterized in that** the catalyst is subject to a photochemical activation from its front and back surfaces.

10. An air purifier **characterized in that** a catalyst having titanium oxide as a main component and activated by noble metal is provided on the surface of the reflecting plate of an illumination lamp.

## INTERNATIONAL SEARCH REPORT

International application No.
PCT/JP00/08340

**A. CLASSIFICATION OF SUBJECT MATTER**
Int.Cl$^7$ B01D53/86, A61L9/00, 9/18

According to International Patent Classification (IPC) or to both national classification and IPC

**B. FIELDS SEARCHED**

Minimum documentation searched (classification system followed by classification symbols)
Int.Cl$^7$ B01D53/86, A61L9/00, 9/18

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)
WPI/L

**C. DOCUMENTS CONSIDERED TO BE RELEVANT**

| Category* | Citation of document, with indication, where appropriate, of the relevant passages | Relevant to claim No. |
|---|---|---|
| EX | JP, 2000-325452, A (Toyo Element Industry Co., Ltd.), 28 November, 2000 (28.11.00), entire description (Family: none) | 1-10 |
| X<br>Y<br>A | JP, 11-300161, A (Nippon Muki Kagaku Kogyo K.K.), 02 November, 1999 (02.11.99), Par. Nos. [0007] to [0011] (Family: none) | 1,2,6,7,9<br>3,8<br>4,5 |
| Y | JP, 2000-167353, A (Denso Corporation), 20 June, 2000 (20.06.00), Par. Nos. [0012] to [0019] (Family: none) | 3,8 |
| X | JP, 11-207148, A (Makii Engineering K.K.), 03 August, 1999 (03.08.99), Claim 1; Figs (Family: none) | 10 |
| X | EP, 903389, A1 (Toto Ltd.), 24 March, 1999 (24.03.99) & AU, 9729777, A & CN, 1226918, A & KR, 2000016116, A & JP, 10-50118, A | 10 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 February, 2001 (19.02.01) | 27 February, 2001 (27.02.01) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP00/08340

C (Continuation). DOCUMENTS CONSIDERED TO BE RELEVANT

| Category* | Citation of document, with indication, where appropriate, of the relevant passages | Relevant to claim No. |
|---|---|---|
| | entire description | |

Form PCT/ISA/210 (continuation of second sheet) (July 1992)

**INTERNATIONAL SEARCH REPORT**

International application No.
PCT/JP00/08340

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

Claims 1-9 describe inventions of an air cleaner equipped with a catalyst, a light source and a power supply, whereas claim 10 describes an invention of an air cleaner having a catalyst provided on the front surface of a reflection sheet of an illuminating light. Therefore, there is no technical relationship among those inventions involving special technical features.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)